# EUROPEAN PATENT APPLICATION

(11) **EP 1 498 414 A1**
(43) Date of publication of application: **19.01.2005**
(21) Application number: 03703248.9
(22) Date of filing: 07.02.2003
(51) Int. Cl.: C07D 301/32, C07D 303/04

(54) **PROCESS FOR PURIFICATION OF PROPYLENE OXIDE**

(30) Priority: 15.02.2002 JP 2002038203
(71) Applicant: Sumitomo Chemical Company, Limited, Tokyo 104-8260 (JP)
(72) Inventor: OKU, Noriaki, Ichihara-shi, Chiba 290-0035 (JP); NAKAYAMA, Toshio, Sodegaura-shi, , Chiba 299-0295 (JP); SHINOHARA, Koji, Ichihara-shi, Chiba 299-0125 (JP)
(74) Representative: Lips, Hendrik Jan George, Ir.
(86) International application number: PCT/JP2003/001288
(87) International publication number: WO 2003/068762

(57) **Abstract**

A process for purifying propylene oxide, which comprises
using a liquid reaction mixture containing propylene oxide, and water, hydrocarbons and oxygen-containing organic compounds as impurities obtained by reacting cumene hydroperoxide with propylene, as a liquid raw material, and
separating and recovering propylene oxide from the liquid raw material, wherein the process comprises the following steps:
first step; a step of subjecting the liquid raw material to extractive distillation using a hydrocarbon of 7 to 10 carbons as an extractant to separate into the overhead stream containing most of water and the oxygen-containing impurities and the bottom stream containing propylene oxide, the hydrocarbons and the extractant,
second step; a step of subjecting the bottom stream to extractive distillation using a hydrocarbon of 7 to 10 carbons as an extractant to separate into the overhead containing propylene oxide and the bottom stream containing hydrocarbons and the extractant, and
third step; subjecting the extractant used in the first step and/or second step to distillation to purify the extractant,
wherein the bottom temperature of the distillation column or extractive distillation column in each of the steps is 130°C or lower.

## Description

### TECHNICAL FIELD

The present invention relates to a process for purifying propylene oxide. More particularly, the present invention relates to a process for purifying propylene oxide by separating and recovering propylene oxide from a liquid raw material which is a liquid reaction mixture containing propylene oxide obtained by reacting cumene hydroperoxide with propylene, wherein the process can effectively obtain high purity propylene oxide.

### BACKGROUND ART

A process for obtaining propylene oxide by reacting cumene hydroperoxide with propylene is publicly known. In this case, oxygen-containing compounds such as water, methanol, acetone and propionaldehyde, hydrocarbons and the like are by-produced in addition to propylene oxide as a target product. Therefore, a purification step of separating and recovering is required for obtaining high purity propylene oxide. It is disclosed in JP05-194455 A to subject propylene oxide containing water, methanol and acetone as impurities to extractive distillation using triethylene glycol as an extractant. In addition, it is disclosed in U.S.Patent 3,338,800 to subject propylene oxide containing water, methyl formate and acetaldehyde as impurities to extractive distillation using a paraffin of 6 to 18 carbons as an extractant. Further, U.S.Patent 5,133,839 discloses a method in which propylene oxide containing water, methanol, methyl formate, propion aldehyde and acetone is purified in multi-stages and an alkane of 8 carbons is used as an extractant.

### DISCLOSURE OF THE INVENTION

An object of the present invention is to provide a process for purifying propylene oxide by separating and recovering propylene oxide from a liquid reaction mixture containing propylene oxide obtained by reacting cumene hydroperoxide with propylene to extraction, wherein the process can effectively obtain high purity propylene oxide.

That is, the present invention relates to a process for purifying propylene oxide, which comprises:
using a liquid reaction mixture containing propylene oxide, and water, hydrocarbons and oxygen-containing organic compounds as impurities obtained by reacting cumene hydroperoxide with propylene, as a liquid raw material, and
separating and recovering propylene oxide from the liquid rawmaterial, wherein the process comprises the following steps :
   first step; a step of subjecting the liquid raw material to extractive distillation using a hydrocarbon of 7 to 10 carbons as an extractant to separate into the overhead stream containing most of water and the oxygen-containing impurities and the bottom stream containing propylene oxide, the hydrocarbons and the extractant,
   second step; a step of subjecting the bottom stream obtained in the first step to extractive distillation using a hydrocarbon of 7 to 10 carbons as an extractant to separate into the overhead stream containing propylene oxide and the bottom stream containing hydrocarbons and the extractant, and
   third step; a step of subjecting the extractant used in the first step and/or the second step to distillation to purify the extractant,
wherein the bottom temperature of a distillation column or extractive distillation column used in each of the steps is 130°C or lower.

### BRIEF EXPLANATION OF THE DRAWING

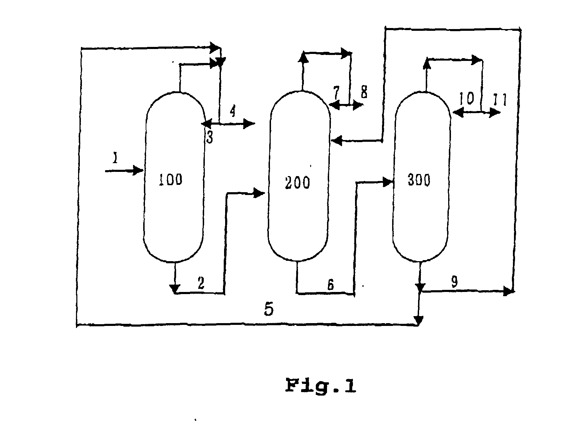

Fig. 1 is a figure showing one example of flow of a purification process of the present invention.

### Explanation of symbols:

100: Extractive distillation column, 200: Extractive distillation column, 300: Distillation column, 1.Line for supplying a propylene oxide raw material liquid containing water, hydrocarbons and oxygen-containing compounds, 2. Bottom stream line of extractive distillation column 100, 3.Refluxing line of extractive distillation column 100, 4. Overhead stream line of extractive distillation column 100, 5.Extractant supplying line of extractive distillation column 100, 6. Bottom stream line of extractive distillation column 200, 7.Refluxing line of extractive distillation column 200, 8. Purifiedpropylene oxide stream line, 9. Extractant supplying line of extractive distillation column 200, 10. Refluxing line of distillation column 300, 11. Overhead stream line of distillation column 300

### BEST MODE FOR CARRYING OUT THE INVENTION

The liquid raw material to be subjected to the purification process of the present invention is a liquid reaction mixture containing propylene oxide obtained by reaction of cumene hydroperoxide with propylene.

Usually, the reaction is carried out in the presence of a catalyst. The reaction is preferably carried out in the presence of a catalyst containing a titanium-containing silicon oxide from the viewpoint of obtaining a desired product under high yield and high selectivity. The catalyst is preferably a so-called titanium-silica catalyst containing titanium chemically bonded to silica. For example, a catalyst in which a titanium compound is supported on silica, a catalyst in which a titanium compound is combined with silica by a co-precipitation method or a sol-gel method, or a zeolite compound containing titanium can be listed.

The reaction of cumene hydroperoxide with propylene can be carried out in a liquid phase using a solvent. The solvent should be liquid under the reaction temperature and pressure, and substantially inert to the reactants and the products. The solvent may be composed of a substance existing in a solution of cumene hydroperoxide used. When, for example, cumene hydroperoxide is a mixture with cumene, it is also possible to substitute cumene for the solvent, without adding any solvent in particular. Other useful solvents include monocyclic aromatic compounds (e.g. benzene, toluene, chlorobenzene. o-dichlorobenzene), alkanes (e.g. octane, decane, dodecane) and the like.

The reaction temperature is usually 10 to 200°C, preferably 25 to 200°C. The pressure may be a pressure enough to keep the reactionmixtureliquid. Inusual, thepressureis advantageously from 100 to 10000 kPa. The reaction can advantageously be carried out using a catalyst in the form of a slurry or a fixed-bed. The fixed-bed is preferred in the case of a large-scale industrial operation. In addition, the reaction can be carried out by a batch process, a semi-continuous process or a continuous process.

The liquid raw material contains propylene oxide, water, hydrocarbons and oxygen-containing organic compounds, as the hydrocarbons, hydrocarbons having 3 to 7 carbon atoms can be listed, and as the oxygen-containing organic compounds, compounds such as methanol, propion aldehyde, acetone and methyl formate can be listed.

One example of a flow of preferable embodiment of the present invention is shown in Fig. 1. A propylene oxide raw material liquid containing water, hydrocarbons and oxygen-containing organic compounds as impurities is obtained by distilling and separating a liquid reaction mixture obtained by reacting propylene with cumene hydroperoxide in the presence of a catalyst. The liquid raw material is fed to an extractive distillation column 100 through a line 1. At the same time, for example, n-haptane as an extractant is fed thereto. The extractive distillation column 100 is a column for removing most of water and oxygen-containing organic compounds contained in the raw material, propylene oxide, they are discharged through a line 4 as the overhead stream, and n-heptane fed through a line 5 and propylene oxide fed through the line 1 are obtained as the bottom stream. The bottom stream of the extractive distillation column 100 is fed to an extractive distillation column 200 through a line 2, n-heptane is simultaneously fed to the extractive distillation column 200 through a line 9. Purified propylene oxide is obtained through a line 8 from the overhead, most of the hydrocarbons contained in the propylene oxide raw material and n-heptane as the extractant are obtained through a line 6 from the bottom. The bottom stream obtained from the extractive distillation column 200 is fed to a distillation column 300 through a line 6, most of the hydrocarbons contained in the propylene oxide raw material is discharged through a line 11, and the bottom stream is recycled to the extractive distillation columns 100 and/or 200 through line 5 and/or line 9, respectively. In the present invention, operations of columns 100, 200 and 300 are carried out at a temperature of the bottom of 130°C or lower, preferably 120°C or lower. When the bottom temperature becomes too high, not only a loss of propylene oxide but also degradation in purity of propylene oxide result from polymerization and/or isomerization of propylene oxide.

### EXAMPLE

Next, the present invention is illustrated by Examples.

### Example 1

Propylene oxide and cumyl alcohol were obtained by reacting cumene hydroperoxide with propylene in the presence of a catalyst. After propylene oxide was separated from unreacted propylene and cumyl alcohol in the liquid reaction mixture by distillation, crude propylene having the following composition was obtained:

| | |
|---|---|
| Water | 0.07% by weight |
| Acetaldehyde | 0.003% by weight |
| Methanol | 0.008% by weight |
| Hydrocarbons | 0.02% by weight |
| The balance | propylene oxide |

Crude propylene oxide having the above-described composition as a raw material was purified.

### First step

The crude propylene oxide having the above-described composition as raw material was fed at a rate of 80g/hr into a distillation column (inner diameter of 30 mm) having bubble cap trays of real plate number of 80, and n-haptane as an extractant was fed at a rate of 200 g/hr through an overhead line at the top of the column. When the pressure was kept at a gauge pressure of 0.13 MPa, the temperature of the top of the column was 66°C, and the temperature of the bottom of the column was 83°C.

### Second step

The bottom stream of the first step was continuously fed into a distillation column (inner diameter of 30 mm) composed of bubble cap trays of real plate number of 60 and a packed layer of 1 m, and n-heptane as an extractant was fed at a rate of 90 g/hr into a place higher in the plate number of the column than that at which the stream was fed. When the pressure was kept at a gauge pressure of 0.1 MPa, the temperature of the top the column was 48°C, and the temperature of the bottom of the column was 117°C.

The composition of propylene oxide obtained by the above-mentioned operation, became as below:

| | |
|---|---|
| Water | 20 ppm by weight |
| Acetaldehyde | 1 ppm by weight or less |
| Methanol | 1 ppm by weight or less |
| Hydrocarbons | 10 ppm by weight or less |
| The balance | propylene oxide |

Propylene oxide of high purity as the above-described composition could be effectively obtained.

### INDUSTRIAL APPLICABILITY

As described above, according to the present invention, there can be provided a process for purifying propylene oxide by separating and recovering propylene oxide from a liquid raw material liquid which is a liquid reaction mixture containing propylene oxide, and water, hydrocarbons and oxygen-containing organic compounds as impurities obtained by reacting cumene hydroperoxide with propylene, wherein the process can effectively obtain high purity propylene oxide.

## Claims

1. A process for purifying propylene oxide, which comprises:
using a liquid reaction mixture containing propylene oxide, and water, hydrocarbons and oxygen-containing organic compounds as impurities obtained by reacting cumene hydroperoxide with propylene, as a liquid raw material, and
separating and recovering propylene oxide from the liquid raw material, wherein the process comprises the following steps:
first step; a step of subjecting the liquid raw material to extractive distillation using a hydrocarbon of 7 to 10 carbons as an extractant to separate into the overhead stream containing most of water and the oxygen-containing impurities and the bottom stream containing propylene oxide, the hydrocarbons and the extractant,
second step; a step of subjecting the bottom stream to extractive distillation using a hydrocarbon of 7 to 10 carbons as an extractant to separate into the overhead containing propylene oxide and the bottom stream containing hydrocarbons and the extractant, and
third step; subjecting the extractant used in the first step and/or second step to distillation to purify the extractant,
wherein the bottom temperature of the distillation column or extractive distillation column in each of the steps is 130°C or lower.

2. The process according to claim 1, wherein the bottom temperature is 120°C or lower.
